**Europäisches Patentamt**

**European Patent Office**

**Office européen des brevets**

(19)

(11) Veröffentlichungsnummer : **0 449 399 B1**

(12)

# EUROPÄISCHE PATENTSCHRIFT

(45) Veröffentlichungstag der Patentschrift :
**19.05.93 Patentblatt 93/20**

(51) Int. Cl.⁵ : **A61K 6/02,** C08K 3/40

(21) Anmeldenummer : **91250085.7**

(22) Anmeldetag : **22.03.91**

(54) **Polymerisierbare Dentalmasse.**

(30) Priorität : **26.03.90 DE 4009602**

(43) Veröffentlichungstag der Anmeldung :
**02.10.91 Patentblatt 91/40**

(45) Bekanntmachung des Hinweises auf die
Patenterteilung :
**19.05.93 Patentblatt 93/20**

(84) Benannte Vertragsstaaten :
**AT CH DE FR GB IT LI SE**

(56) Entgegenhaltungen :
**EP-A- 0 091 990**
**DE-A- 2 454 101**
**DE-A- 2 736 891**
**Holleman-Wiberg 81-90 Auflage, 1976, S. 568**
**ff.**

(73) Patentinhaber : **IVOCLAR AG**
**Postfach, Bendererstrasse 2**
**FL-9494 Schaan (LI)**

(72) Erfinder : **Rheinberger, Volker, Dr.**
**Floraweg 3**
**FL-9490 Vaduz (LI)**
Erfinder : **Salz, Ulrich, Dr.**
**Rosenweg 2**
**W-8995 Weissenberg (DE)**
Erfinder : **Grabher, Kurt**
**Drewesstrasse 6**
**A-6800 Feldkirch (AT)**

(74) Vertreter : **UEXKÜLL & STOLBERG**
**Patentanwälte**
**Beselerstrasse 4**
**W-2000 Hamburg 52 (DE)**

**Beschreibung**

Die Erfindung betrifft Dentalmassen mit alkalisierender Wirkung wie z.B. Zemente, Kavitätenliner und Wurzelfüllmaterialien, insbesondere eine als Unterfüllungsmaterial geeignete polymerisierbare Dentalmasse auf Basis von mindestens einem polymerisierbaren Monomer oder Prepolymer, einem Härtungskatalysator und anorganischen Füllstoffen.

Unterfüllungsmaterialien dienen bei der Behandlung von Zahnkavitäten zur Überkappung der Pulpa, bevor die eigentliche Füllung gelegt wird. Es ist bekannt, daß Calciumhydroxid für die Bildung von sekundärem Dentin über der Pulpa verantwortlich ist und daß ein umso besserer Schutz erzielt wird, je dicker und fester diese Schicht ist. Die üblicherweise verwendeten Calciumhydroxid enthaltenden Zemente, welche auf Chelatbasis härten, ergeben jedoch keine befriedigende Festigkeit.

In der EP-A-189 903 wird ein Unterfüllungsmaterial mit Gehalt an Calciumhydroxid oder einem Calciumhydroxid bildenden Mittel wie Calciumoxid beschrieben, welches photopolymerisierbar ist. Dieses Unterfüllungsmaterial enthält ethylenisch ungesättigte Verbindungen, insbesondere Vinylverbindungen, sowie einen Photokatalysator, z.B. Campherchinon in Kombination mit einem Amin. Ein grundsätzliches Problem besteht jedoch darin, daß Calciumhydroxid bzw. Calciumoxid sehr opak sind, so daß nur geringe Aushärtungstiefen erreicht werden können, wie Versuche mit einem Handelsprodukt gemäß der EP-A-189 903 Prisma VLC Dycal® der Fa. Dentsply International, Inc.) zeigen.

Mit der Erfindung soll ebenfalls ein Unterfüllungsmaterial bereitgestellt werden, welches hydrolyse- und säurebeständig ist, geringe Wasserlöslichkeit aufweist, eine gute Druckfestigkeit zeigt und nicht toxisch ist. Darüber hinaus soll eine kontrollierte Abgabe von Calciumhydroxid erreicht werden, um gegenüber der Pulpa eine alkalisierende Wirkung zu erreichen und diese gegen Säuren und bakterielle Angriffe zu schützen. Insbesondere soll das Material nach der Polymerisation eine größere Aushärtungstiefe und eine geringere Schrumpfung aufweisen.

Es wurde überraschend gefunden, daß sich diese Aufgabe dadurch lösen läßt, daß man in die Dentalmasse als Füllstoff ein Glaspulver mit hohem Gehalt an Calciumoxid einarbeitet. Durch Wasseraufnahme werden $Ca^{2+}$- und $OH^-$-Ionen freigesetzt. Die erfindungsgemäß verwendeten Glaspulver sind nicht opak und behindern daher die Lichthärtung der Dentalmasse nicht.

Das erfindungsgemäß eingesetzte Glaspulver enthält neben 40 bis 75 Gew.% Calciumoxid noch 5 bis 30 Gew.% Boroxid und 5 bis 35 Gew.% Siliciumdioxid. Eine bevorzugte Zusammensetzung besteht aus 45 bis 60 Gew.%. Calciumoxid, 15 bis 28 Gew.%. Boroxid und 10 bis 30 Gew.% Siliciumdioxid. Die durchschnittliche Teilchengröße (Gewichtsmittel) der Glaspulver liegt vorzugsweise zwischen etwa 10 und 30 μm, geeignet sind jedoch Pulver mit einer Teilchengröße von 1 bis 100 μm.

Wie oben bereits ausgeführt, eignen sich die erfindungsgemäßen Dentalmassen aufgrund der gesteuerten Freisetzung von Calciumionen aus dem Glaspulver besonders als Unterfüllungsmaterial. Vorzugsweise handelt es sich um lichthärtende Einkomponenten-Massen. Diese enthalten neben dem Glaspulver ggf. weitere anorganische Füllstoffe sowie mindestens ein polymerisierbares Monomer oder Prepolymer und geeignete Katalysatoren.

Gegebenenfalls kann das Glas außerdem Oxide, z.B. Kaolin oder Oxide von Sr, Ba, La, Zr oder Seltenen Erden enthalten. Ca-Wolframat, Ba-Wolframat sowie fluor- und/oder phosphorhaltige Verbindungen wie z.B. NaF, KF, $BaF_2$, $SrF_2$, Fluoride Seltener Erden, Ca- oder Al-Phosphat und Kryolith sind weitere geeignete Zusätze.

Als besonders geeignete anorganische Füllstoffe haben sich die Fluoride der Seltenen Erdmetalle (SE) mit den Ordnungszahlen 59 bis 71 erwiesen, wobei bevorzugte Verbindungen diejenigen der Elemente 66 bis 71 sind.

Vorzugsweise wird Ytterbiumfluorid verwendet. Die Fluoride der Seltenen Erdmetalle werden im allgemeinen als Pulver in den Dentalwerkstoff eingearbeitet. Die mittlere Korngröße der Primärteilchen kann dabei schwanken. Bei einem mikrogefüllten Zahnfüllungsmaterial liegt sie im Bereich von 5 bis 700, insbesondere 20 bis 500, vorzugsweise 50 bis 300 nm. Gegebenenfalls kann die mittlere Primärteilchengröße auch im Bereich von 700 nm bis 15 μm liegen.

Der Gehalt an SE-Fluoriden, bezogen auf das Gesamtgewicht, beträgt zwischen 1 und 50 %, insbesondere 5 bis 40 % ; vorzugsweise liegt er zwischen 10 und 30 % . Er hängt insbesondere von der gewünschten Röntgenopazität bzw. Transparenz ab. Es können auch Mischungen der SE-Fluoride verwendet werden.

Zusätzlich können noch andere Füllstoffe wie Ba-, Sr-, Ca-, Li-Al-Silikatgläser sowie $BaSO_4$, $CaWO_4$, Bi-Subnitrat und Bi-Carbonat eingesetzt werden.

Im Dentalwerkstoff sind üblicherweise noch andere, nicht röntgenopake, anorganische Bestandteile vorhanden. Als Füllstoffe eignen sich z.B. amorphe Kieselsäuren, insbesondere pyrogene oder gefällte Kieselsäure mit einer BET-Oberfläche von etwa 20 bis 400 m²/g. Insbesondere werden pyrogene Kieselsäuren mit einer BET-Oberfläche von 30 bis 300 m²/g und einer mittleren Korngröße der Primärteilchen von etwa 5 bis 50 nm verwendet, wobei speziell bevorzugte Materialien im Bereich zwischen 1 und 50 nm liegen. Kieselsäuren mit einer mittleren Primärteilchengröße von 50 bis 1000, vorzugsweise 100 bis 300 nm können jedoch ebenfalls Verwendung finden. Li-Al-Silikatgläser eignen sich wie oben erwähnt ebenfalls.

Die Menge der nicht röntgenopaken Füllstoffe im Dentalwerkstoff ist abhängig von der Menge der verwendeten SE-Fluoride und bewegt sich im allgemeinen im Bereich von 5 bis 84 %, insbesondere 10 bis 70 %, vorzugsweise 20 bis 50 %. Insgesamt beträgt der Gehalt an Füllstoffen (Glaspulver, SE-Fluoriden und weiteren anorganischen Verbindungen) 6 bis 85, vorzugsweise 15 bis 85 und insbesondere 30 bis 85 Gew. % .

Die anorganischen Bestandteile des Dentalwerkstoffes können in üblicher Weise silanisiert sein, um den Verbund zwischen organischer Matrix und anorganischem Füllstoff zu verbessern. Als Haftvermittler eignet sich z.B. 3-Methacryloxypropyltrimethoxysilan. Die Menge des eingesetzten Haftvermittlers richtet sich nach der Art und der spezifischen Oberfläche des Füllstoffes und der gewünschten Viskosität des Dentalwerkstoffes.

Der Dentalwerkstoff muß ferner eine polymerisierbare Vinylverbindung enthalten. Insbesondere eignen sich hierfür monofunktionelle oder polyfunktionelle (Meth)acrylate, die allein oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Tetraethylenglycoldimethacrylat, Ethylenglycoldimethacrylat, Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, ethoxyliertes Bisphenol-A-dimethacrylat, aber auch Bis-GMA (2,2-bis-4-(3-methacryloxy-2-hydroxypropyl)-phenylpropan) sowie die Reaktionsprodukte aus Isocyanaten, insbesondere Di- und-/oder Triisocyanaten und OH-gruppenhaltigen Methacrylaten in Frage. Beispiele dafür sind die Umsetzungsprodukte von 1 Mol Hexamethylendiisocyanat mit 2 Mol 2-Hydroxyethylmethacrylat, von 1 Mol Tri(6-isocyanatohexyl)biuret mit 3 Mol Hydroxyethylmethacrylat und von 1 Mol Trimethylhexamethylendiisocyanat mit 2 Mol Hydroxyethylmethacrylat, die im folgenden als Urethandimethacrylat bezeichnet werden. Der Anteil dieser meist langkettigen Verbindungen im Dentalwerkstoff bewegt sich zwischen 10 und 50 Gew. %. Im Prinzip kommen alle für einen Dentalwerkstoff brauchbaren Bindemittel in Frage.

Der Dentalwerkstoff kann je nach Art des verwendeten Katalyators heiß, kalt oder durch Photopolymerisation aushärtbar sein. Kombinationen daraus sind jedoch auch möglich (Dualhärtung).

Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch $\alpha,\alpha$'-Azo-bis-(isobutyroethylester), Benzpinakol und 2,2'-Dimethylbenzpinakol sind geeignet.

Als Katalysatoren für die Photopolymerisation können z.B. Benzophenon und seine Derivate, Acylphosphinoxide sowie Benzoin und seine Derivate verwendet werden. Beispiele für bevorzugte Photoinitiatoren sind die $\alpha$-Diketone wie 9,10-Phenanthrenchinon, Diacetyl, Furil, Anisil, 4,4'-Dichlorbenzil und 4,4'-Dialkoxybenzil. Campherchinon wird besonders bevorzugt verwendet. Die Verwendung der Photoinitiatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin und N,N-Dimethyl-sym.-xylidin, N,N-Dimethyl-p-toluidin und p-Dimethylaminobenzoesäureethylester. Als Katalysatorgemische können die Photoinitiatoren und Reduktionsmittel zusammen mit Katalysatoren für die Heißpolymerisation (bevorzugt mit Peroxiden) eingesetzt werden.

Als Katalysatoren für die Kaltpolymerisation werden Radikale liefernde Systeme, z.B. Benzoyl- bzw. Lauroylperoxid zusammen mit Aminen wie N,N,-Dimethyl-sym.-xylidin, N,N-Di-2-hydroxyethyl-p-toluidin oder N,N-Dimethyl-p-toluidin verwendet.

Die Menge dieser Katalysatoren im Dentalwerkstoff liegt üblicherweise zwischen O,1 bis 5 Gew.%.

Dem Dentalwerkstoff können ferner feinteilige Splitter- oder Perlpolymerisate einverleibt werden, die Homo- oder Copolymere der schon beschriebenen Vinylverbindungen sein können. Diese Homo- bzw. Copolymeren können ihrerseits mit den beschriebenen anorganischen Füllstoffen, auch den röntgenopaken, gefüllt sein. Es wird dazu auf die EP-PS 11 190 und die DE-PS 24 03 211 verwiesen . Ferner kann der Dentalwerkstoff die üblichen Pigmentierungsmittel und Stabilisatoren enthalten.

Um den Füllungsgrad solcher Füllungsmaterialien zu erhöhen, ist es üblich, z.B. aus Bis-GMA, Triethylenglycoldimethacrylat, dem Glaspulver und gegebenfalls weiteren anorganischen Füllstoffen wie Ytterbiumfluorid und pyrogener Kieselsäure ein Copolymer herzustellen, dieses als Splitterpolymerisat zu vermahlen und dann in das Füllungsmaterial einzuarbeiten.

Bei lichthärtenden Materialien erfolgt die Polymerisation nach dem Legen der Unterfüllung mit einer handelsüblichen Halogenlampe.

Füllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z.B. Benzoylperoxid und in die andere Paste z.B. N,N-Dimethyl-p-toluidin eingemischt. Durch Vermischen etwa gleicher Teile der beiden Pasten erhält man ein Zahnfüllungsmaterial, welches in wenigen Minuten aushärtet.

Die nachfolgenden Beispiele dienen der Erläuterung der Erfindung.

**Beispiele**

Zur Herstellung der erfindungsgemäß als Füllstoff eingesetzten Gläser wurden die Rohstoffe in einer Kugelmühle miteinander gemischt, die Mischungen wurden anschließend in einem Schmelztiegel aus Aluminiumoxid bei 1000°C eine Stunde lang gesintert. Die vorgesinterten Mischungen wurden nochmals homogenisiert. Anschließend wurden die Mischungen in einem Platintiegel geschmolzen (30 Min. bei 1500°C). Die Schmelzen wurden in Wasser abgeschreckt, die Masse wurde getrocknet und schließlich in einer Kugelmühle trocken vermahlen. Als Rohstoffe eignen sich Calciumcarbonat, Quarz und Boroxid. Gegebenenfalls werden geringe Mengen von bis zu 5 Gew.% eines Flußmittels wie z.B. Kryolith, NaF, KF etc. zugefügt. Der Calciumcarbonat-Anteil liegt so hoch, daß im Glas das Verhältnis von $CaO : SiO_2$ normalerweise über 1,5 beträgt.

Die Gläser wurden mit einer polymerisierbaren Vinylverbindung (Monomer) homogen gemischt. Einigen Mischungen wurden noch andere anorganische Substanzen beigemischt. Das Monomer für die Versuche A bis F und H bis K war ein Gemisch aus 2,2-bis[p-(β-Hydroxyethoxy)phenyl]propan-dimethacrylat (SR 348) und einem Urethandimethacrylat (RM3) (Reaktionsprodukt von Trimethylhexamethylendiisocyanat und Hydroxyethylmethacrylat). Das Monomer von Versuch G war eine Mischung von 56,6 g 2,7,7,9,15-Pentamethyl-4,13-dioxo-3, 14-dioxa-5, 12-diaza-hexadecan-1,16-diyl-dimethacrylat und 5 g 3,6-Dioxaoctamethylendimethacrylat. Der Vergleichsversuch G wurde mit dem Verkaufsprodukt Prisma VLC Dycal® der Firma De Trey Dentsply gemacht. Versuch A ist ebenfalls ein Vergleichsversuch nach dem Stand der Technik.

Als Photokatalysatoren dienten Campherchinon (CQ) und Cyanoethylmethylanilin (CEMA). Das Bindemittel für die Versuche A bis F und H bis K hatte folgende Zusammensetzung:

SR 348    80,0 Gew.%
RM3        19,17 Gew.%
CQ          0,3 Gew.%
CEMA      0,5 Gew.%
BHT        0,03 Gew.%
BHT = 2,6-di-tert.-Butyl-p-kresol. (Stabilisator)

Für die Messung der Aushärtungstiefe, Transparenz, Druckfestigkeit, Wasserlöslichkeit, Wasseraufnahme und $Ca(OH)_2$-Abgabe wurden Prüfkörper hergestellt. Die Herstellung der Prüfkörper und ihre Abmessungen:

Aushärtungstiefe - Stahlform Durchmesser 4 mm, Höhe 6 mm. Die Form wird gefüllt und während 40 Sekunden mit einem handelsüblichen Lichthärtungsgerät (Heliomat® der Firma Vivadent) belichtet.

Transparenz - der Prüfkörper wird in einer Stahlform mit den Abmessungen 15 x 9 x 1 mm hergestellt und während 3 Minuten in einem Lichthärtungsgerät unter 6 bar Druck ausgehärtet. Die Messung der Transparenz erfolgte mit einem Gerät, wie es in der EP-A-189 540, Beispiel 1 beschrieben ist.

Druckfestigkeit - Prüfkörper werden in einer Delrinform mit einem Durchmesser von 4 mm und einer Höhe von 6 mm hergestellt. Das Material wird während 3 Minuten mit dem Lichthärtungsgerät ausgehärtet.

Wasserlöslichkeit und Wasseraufnahme - In einer Stahlform mit 15 mm Durchmesser und einer Höhe von 0,5 mm wird der Prüfkörper hergestellt. Die Aushärtung geschieht wie oben beschrieben.

Die Versuchsergebnisse sind aus den Tabellen I und II ersichtlich.

Diskussion der Versuchsergebnisse:

Die Versuche zeigen für die erfindungsgemäßen Unterfüllungsmaterialien bezüglich Durchhärtungstiefe, Transparenz und Druckfestigkeit etwa die gleichen Werte, wie sie mit $Ca(OH)_2$ als Füllstoff erhalten werden (Versuch A). Dem Verkaufsprodukt Prisma VLC Dycal® (Versuch G) sind die erfindungsgemäßen Unterfüllungsmaterialien in den meisten Eigenschaften überlegen.

Die erfindungsgemäßen Produkte sind in ihrer Wasserunlöslichkeit bzw. Wasseraufnahme und insbesondere in der Abgabe von Calciumhydroxid deutlich besser.

Tabelle I

| | A | B | C | D | E | F | G |
|---|---|---|---|---|---|---|---|
| Monomer | 30% | 35% | 30% | 30% | 20% | 25% | 61,6% |
| CaO | | 45,0 | 59,4 | 55,2 | 45,0 | 45,0 | |
| $B_2O_3$ Glas | | 26,5 40% | 25,6 70% | 21,7 70% | 26,5 50% | 26,5 50% | |
| $SiO_2$ | | 28,5 | 15,0 | 23,1 | 28,5 | 28,5 | |
| $YbF_3$ | | 25% | | | | | |
| $BaSO_4$ | 20% | | | | | | 18,9 % |
| Ba-Glas | | | | | 30% | | |
| Aerosil OX50 | | | | | | 25% | |
| $Ca(OH)_2$ | 50% | | | | | | 18,9 % |
| Durchhärtungstiefe mm | 3,4±0,1 | 3,1±0,1 | 2,7±0,1 | 3,1±0,1 | 3,5±0,2 | 2,7±0,1 | 1,8±0,1 |
| Transparenz % | 28±1 | 29±1 | 19±1 | 21±1 | 25±1 | 19±1 | 21±1 |
| Druckfestigkeit $N/mm^2$ | | | | | | | |
| nach 24 h | 137±2 | 173±7 | 119±3 | 117±8 | 224±13 | 254±6 | 180±8 |
| nach 1 W | 161±8 | 174±8 | 125±5 | 105±10 | 242±12 | 253±18 | 190±9 |
| Verformung bei 200N | | | | | 7±0,2% | 9±1% | |
| Biegefestigkeit $N/mm^2$ | | | | | 75±7 | | 29±4 |
| Biegemodul $N/mm^2$ | | | | | 8500±500 | | 910±170 |
| $H_2O$ Löslichkeit n. 24h | 1,2±0,3 | 0,15±0,05 | 0,75±0,04 | 0,8±0,3 | 0,1±0,05 | 0,1±0,04 | 0,25±0,1 |
| $H_2O$ Aufn. n. 24h | 1,3±0,3 | 0,6±0,1 | 0,2±0,06 | 0,35±0,1 | 0,3±0,05 | 0,3±0,05 | 2,3±0,25 |

EP 0 449 399 B1

Tabelle II

| Ca(OH)$_2$ Abgabe (ppm) nach | H | J | K | G |
|---|---|---|---|---|
| Monomer | 30% | 30% | 30% | s.Tab.1 |
| CaO | 59,4 | 59,4 | 55,2 | |
| B$_2$O$_3$ Glas | 19,6 70% | 25,6 70% | 21,7 70% | |
| SiO$_2$ | 21,0 | 15,0 | 23,1 | |
| 1 Tg | 101,3 | 154,6 | 182,7 | 34,0 |
| 2 Tg | 238,4 | 289,3 | 277,5 | 61,0 |
| 3 Tg | 307,2 | 379,2 | 324,8 | 74,6 |
| 4 Tg | 343,7 | 424,8 | 370,2 | 86,4 |
| 11 Tg | 431,4 | 550,5 | 501,5 | 114,0 |
| 25 Tg | 502,9 | 631,1 | 575,6 | 115,0 |
| 8 W | 596,9 | 744,6 | 681,6 | 119,5 |

Je zwei Prüfkörper ($\emptyset$ 20 mm, h = 1 mm) werden in 50 ml H$_2$O dest. eingehängt. Nach verschiedenen Zeitabständen wurde die Lösung mit 0,01 HCl zurücktitriert und der Verbrauch als Ca(OH)$_2$ berechnet. Die Proben wurden bei 37°C gelagert. Nach jeder Messung wurde das H$_2$O erneuert. Die Ca(OH)$_2$ Abgabe ist kumulativ angegeben.

## Patentansprüche

1. Als Unterfüllungsmaterial geeignete polymerisierbare Dentalmasse auf Basis von mindestens einem polymerisierbaren Monomer oder Prepolymer, einem Härtungskatalysator und anorganischen Füllstoffen, **dadurch gekennzeichnet**, daß die Masse als Füllstoff ein Glaspulver mit einem Gehalt an
40 - 75 Gew.% CaO
5 - 30 Gew.% B$_2$O$_3$
5 - 35 Gew.% SiO$_2$
enthält.

2. Dentalmasse nach Anspruch 1, **dadurch gekennzeichnet**, daß die Masse als Füllstoff ein Glaspulver mit einem Gehalt an
45 - 60 Gew.% CaO
15 - 28 Gew.% B$_2$O$_3$
10 - 30 Gew.% SiO$_2$
enthält.

3. Dentalmasse nach Anspruch 1 oder 2, **dadurch gekennzeichnet**, daß die Masse als Füllstoff ein Glaspulver enthält, welches im wesentlichen aus CaO, B$_2$O$_3$ und SiO$_2$ besteht.

4. Dentalmasse nach Anspruch 1 bis 3, **dadurch gekennzeich**net, daß die Masse weitere anorganische Füllstoffe enthält.

5. Dentalmasse nach Anspruch 4, **dadurch gekennzeichnet**, daß die Masse Ytterbiumfluorid und/oder Bariumsulfat und/oder Kieselsäure und/oder röntgenopake und/oder nicht röntgenopake Dentalgläser als weitere anorganische Füllstoffe enthält.

6. Dentalmasse nach Anspruch 1 bis 5, **dadurch gekennzeichnet**, daß die Masse lichthärtend ist.

7. Dentalmasse nach Anspruch 6, **dadurch gekennzeichnet**, daß die Masse als Photokatalysator Campherchinon in Kombination mit einem Amin enthält.

8. Dentalmasse nach Anspruch 1 bis 7, **dadurch gekennzeichnet**, daß die Masse als photopolymerisierbare Monomere Vinylverbindungen in einer Menge von 10 bis 50 Gew. % enthält.

9. Glaspulver zur Verwendung als Füllstoff in Dentalwerkstoffen, **gekennzeichnet durch** einen Gehalt an
40 - 75 Gew.% CaO
5 - 30 Gew.% $B_2O_3$
5 - 35 Gew.% $SiO_2$.
aufweist.

10. Glaspulver nach Anspruch 9, **dadurch gekennzeichnet**, daß es
45 - 60 Gew.% CaO
15 - 28 Gew.% $B_2O_3$
10 - 30 Gew.% $SiO_2$
enthält.

## Claims

1. Polymerisable dental material suitable as underfilling material based on at least one polymerisable monomer or prepolymer, a curing catalyst and inorganic fillers, characterised in that the material comprises, as filler, a glass powder containing
40 - 75 wt-% CaO
5 - 30 wt-% $B_2O_3$
5 - 35 wt-% $SiO_2$.

2. Dental material according to claim 1, characterised in that the material comprises, as filler, a glass powder containing
45 - 60 wt-% CaO
15 - 28 wt-% $B_2O_3$
10 - 30 wt-% $SiO_2$.

3. Dental material according to claim 1 or 2, characterised in that the material comprises, as filler, a glass powder which essentially consists of CaO, $B_2O_3$ and $SiO_2$.

4. Dental material according to claims 1 to 3, characterised in that the material comprises other inorganic fillers.

5. Dental material according to claim 4, characterised in that the material comprises ytterbium fluoride and/or barium sulphate and/or silica and/or X-ray-opaque and/or non-X-ray-opaque dental glasses as other inorganic fillers.

6. Dental material according to claims 1 to 5, characterised in that the material is light curable.

7. Dental material according to claim 6, characterised in that the material comprises, as photocatalyst, camphorquinone in combination with an amine.

8. Dental material according to claims 1 to 7, characterised in that the material comprises, as photopolymerisable monomers, vinyl compounds in a quantity of 10 to 50 wt-%.

9. Glass powder for use as filler in dental materials, characterised by comprising
40 - 75 wt-% CaO
5 - 30 wt-% $B_2O_3$
5 - 35 wt-% $SiO_2$.

10. Glass powder according to claim 9, characterised in that it contains
45 - 60 wt-% CaO
15 - 28 wt-% $B_2O_3$
10 - 30 wt-% $SiO_2$.

**Revendications**

1. Pâte dentaire polymérisable, convenant comme matière de remplissage partiel, à base d'au moins un monomère ou prépolymère polymérisable, d'un catalyseur de durcissement et de matières de charge inorganiques, caractérisée en ce qu'elle comporte, comme matière de charge, une poudre de verre contenant
40 à 75% en poids de CaO,
5 à 30% en poids de $B_2O_3$,
5 à 35% en poids de $SiO_2$.

2. Pâte dentaire selon la revendication 1, caractérisée en ce que la pâte comporte, comme matière de charge, une poudre de verre contenant
45 à 60% en poids de CaO,
15 à 28% en poids de $B_2O_3$,
10 à 30% en poids de $SiO_2$.

3. Pâte dentaire selon la revendication 1 ou 2,
caractérisée en ce que la pâte comporte, comme matière de charge, une poudre de verre, laquelle est essentiellement constituée de CaO, de $B_2O_3$ et de $SiO_2$.

4. Pâte dentaire selon les revendications 1 à 3,
caractérisée en ce que la pâte contient d'autres matières de charge inorganiques.

5. Pâte dentaire selon la revendication 4, caractérisée en ce que la pâte contient, comme matières de charge supplémentaires, du fluorure d'ytterbium et/ou du sulfate de baryum et/ou de l'acide silicique et/ou des verres dentaires opaques et/ou non opaques aux rayons X.

6. Pâte dentaire selon les revendications 1 à 5,
caractérisée en ce que la pâte est photodurcissable.

7. Pâte dentaire selon la revendication 6, caractérisée en ce que la pâte contient, comme photocatalyseur, de la camphroquinone combinée à une amine.

8. Pâte dentaire selon les revendications 1 à 7,
caractérisée en ce que la pâte contient, comme monomères photopolymérisables, des composés vinyliques en une quantité valant de 10 à 50% en poids.

9. Poudre de verre à utiliser comme matière de charge dans des produits dentaires, caractérisée en ce qu'elle renferme
40 à 75% en poids de CaO,
5 à 30% en poids de $B_2O_3$,
5 à 35% en poids de $SiO_2$.

10. Poudre de verre selon la revendication 9, caractérisée en ce qu'elle renferme
45 à 60% en poids de CaO,
15 à 28% en poids de $B_2O_3$,
10 à 30% en poids de $SiO_2$.